(19) Europäisches Patentamt
European Patent Office
Office européen des brevets

(11) **EP 4 651 081 A2**

## (12) EUROPEAN PATENT APPLICATION

(43) Date of publication:
**19.11.2025 Bulletin 2025/47**

(21) Application number: **25201473.3**

(22) Date of filing: **06.09.2024**

(51) International Patent Classification (IPC):
*G06T 7/33* (2017.01)

(52) Cooperative Patent Classification (CPC):
**G06T 7/33; A61B 6/469; A61B 6/502; A61B 8/5207; G06T 7/0012; G06T 7/73; G16H 30/40;** A61B 6/563; G06T 2207/10116; G06T 2207/20096; G06T 2207/20101; G06T 2207/20104; G06T 2207/30068

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **06.09.2023 US 202318461695**

(62) Document number(s) of the earlier application(s) in accordance with Art. 76 EPC:
**24198933.4 / 4 521 346**

(71) Applicant: **FUJIFILM Healthcare Americas Corporation**
**Lexington, MA 02421 (US)**

(72) Inventors:
• **MORITA, Keiichi**
**Cary, 27519 (US)**
• **MINNICH, Jeffrey**
**Milton, 30004 (US)**
• **ROGERS, Jeanmarie**
**Portsmouth, 02871 (US)**

(74) Representative: **Dehns**
**10 Old Bailey**
**London EC4M 7NG (GB)**

(54) **METHODS AND SYSTEMS FOR MANIPULATING MAMMOGRAMS**

(57) A method (1000) for marking a region of interest (110) in a mammogram (10), comprising identifying (1020) a first posterior nipple line (114E) in the first mammogram (10E); identifying (1030) a region of interest (110A) in the first mammogram; identifying (1040) a reference line (117A) in the first mammogram, the reference line being perpendicular to the first posterior nipple line and extending from the posterior nipple line through the region of interest; calculating (1050) a distance, d, the distance d extending from the first nipple tip to the reference line along the first posterior nipple line; identifying (1070) a second posterior nipple line (114F) in the second mammogram (10F); and providing (1080), on the second mammogram, a region-of-interest marker (115A), the region-of-interest marker extending perpendicular from the second posterior nipple line the distance d from the second nipple tip along the second posterior nipple line.

FIG. 3

EP 4 651 081 A2

**Description**

The present invention relates to systems and methods for manipulating images, and more specifically, mammograms. The systems and methods described herein can mark regions of interest and align mammograms utilizing a posterior nipple line.

In medical imaging, Picture Archiving and Communication Systems ("PACS") are a combination of computers and networks dedicated to the storage, retrieval, presentation, and distribution of images. While medical information can be stored in a variety of formats, a common format of image storage is DICOM. DICOM is a standard in which, among other things, medical images and associated meta-data can be communicated from imaging modalities (e.g., x-ray (or x-rays' digital counterparts: computed radiography ("CR") and digital radiography ("DR")), computed tomography ("CT"), and magnetic resonance imaging ("MRI") apparatuses) to remote storage and/or client devices for viewing and/or other use.

When viewing medical images, for example, mammogram images stored in DICOM format, it can be important for a user to identifying corresponding regions of interest in two different images. For example, when viewing a single breast imaged by two different projections (such as a craniocaudal (CC) view and a mediolateral oblique view (MLO)), a user can be interested in identifying a region of interest in the first image, and automatically having a corresponding region of interest marked in the second image. Typically, this is performed by determining a distance from a reference, such as the nipple. For example, arched reference lines having a center point on the nipple can be drawn on each image. Arched lines with similar radii drawn in two different images can be interpreted as the same distance from the nipple in the respective images. However, such a process is imperfect at least because the CC and MLO views are captured from different angles, the x rays are taken along straight lines, not arcs, and the breast can be compressed in the CC image.

Additionally, it can be beneficial for a user to be able to register two images. For example, when comparing past and current studies, a user needs images to be aligned. Conventional methods for alignment include pixel matching. However, such alignment can be inefficient, challenging, and imperfect.

Accordingly, there is a need for improvement methods and systems for marking regions of interest mammograms and registering mammograms.

The purposes and advantages of the invention will be set forth in and apparent from the description that follows, as well as will be learned by practice of the invention. Additional advantages of the invention will be realized and attained by the methods and systems particularly pointed out in the written description and claims hereof, as well as the appended figures.

To achieve these and other advantages and in accordance with the purpose of the invention, as embodied and broadly described, the invention is directed to systems and methods for marking a region of interest in a mammogram.

According to a first aspect, the present invention provides a method for marking a region of interest in a mammogram, comprising: receiving a first mammogram of a patient, the first mammogram comprising a first nipple tip and being a first projection; identifying a first posterior nipple line in the first mammogram; identifying a region of interest in the first mammogram; identifying a reference line in the first mammogram, the reference line being perpendicular to the first posterior nipple line and extending from the first posterior nipple line through the region of interest; calculating a distance, d, the distance d extending from the first nipple tip to the reference line along the first posterior nipple line; receiving a second mammogram of the patient, the second mammogram comprising a second nipple tip and being a second projection; identifying a second posterior nipple line in the second mammogram; and providing, on the second mammogram, a region-of interest-marker, the region-of-interest marker extending perpendicular from the second posterior nipple line the distance d from the second nipple tip along the second posterior nipple line.

The first posterior nipple line can be perpendicular to a chest wall. The method can include receiving a user input identifying the chest wall. The second posterior nipple line can be perpendicular to a chest wall. The method can include receiving a user input identifying the chest wall. The first projection can be one of a craniocaudal view and a mediolateral oblique view. The second projection can be the other of the craniocaudal view and the mediolateral oblique view.

The method can include receiving a user input indicating a location of the first nipple tip. The method can include receiving a user input indicating a location of the second nipple tip. According to a second aspect, the invention provides a system comprising: one or more processors; and a non-transitory memory coupled to the processors comprising instructions executable by the processors. The processors can be operable when executing the instructions to: receive a first mammogram of a patient, the first mammogram comprising a first nipple tip and being a first projection; identify a first posterior nipple line in the first mammogram; identify a region of interest in the first mammogram; identify a reference line in the first mammogram, the reference line being perpendicular to the first posterior nipple line and extending from the first posterior nipple line through the region of interest; calculate a distance, d, the distance d extending from the first nipple tip to the reference line along the first posterior nipple line; receive a second mammogram of the patient, the second mammogram comprising a second nipple tip and being a second projection; identify a second posterior nipple line in the second mammogram; and provide, on the second mammogram, a region-of-interest marker, the region-of-interest marker extending perpendicular from the second posterior nipple line the distance d from the second nipple tip along the second posterior nipple line.

According to a third aspect, the invention provides a method for registering two mammogram images, comprising: receiving a first mammogram of a patient, the first mammogram comprising a first nipple tip having a first vertical coordinate and a first horizontal coordinate; identifying a first posterior nipple line in the first mammogram, the first posterior nipple line having a first angle; receiving a second mammogram of the patient, the second mammogram comprising a second nipple tip having a second vertical coordinate and a second horizontal coordinate; identifying a second posterior nipple line in the second mammogram, the second posterior nipple line having a second angle; shifting the second mammogram vertically by a first difference between the second vertical coordinate and the first vertical coordinate; shifting the second mammogram horizontally by a second difference between the second horizontal coordinate and the first horizontal coordinate; and rotating the second mammogram by a third difference between the second angle and the first angle.

The first posterior nipple line may be perpendicular to a chest wall. The method may comprise receiving a user input identifying the chest wall.

The second posterior nipple line may be perpendicular to a chest wall. The method may comprise receiving a user input identifying the chest wall.

The first mammogram may comprise a craniocaudal view and the second mammogram may comprise a craniocaudal view. The first mammogram may comprise a mediolateral oblique view and the second mammogram may comprise a mediolateral oblique view.

The method may comprise receiving a user input indicating a location of the first nipple tip. The method may comprise receiving a user input indicating a location of the second nipple tip.

According to a fourth aspect, the invention provides a system comprising: one or more processors; and a non-transitory memory coupled to the processors comprising instructions executable by the processors. The processors are operable when executing the instructions to: receive a first mammogram of a patient, the first mammogram comprising a first nipple tip having a first vertical coordinate and a first horizontal coordinate; identify a first posterior nipple line in the first mammogram, the first posterior nipple line having a first angle; receive a second mammogram of the patient, the second mammogram comprising a second nipple tip having a second vertical coordinate and a second horizontal coordinate; identify a second posterior nipple line in the second mammogram, the second posterior nipple line having a second angle; shift the second mammogram vertically by a first difference between the second vertical coordinate and the first vertical coordinate; shift the second mammogram horizontally by a second difference between the second horizontal coordinate and the first horizontal coordinate; and rotate the second mammogram by a third difference between the second angle and the first angle. Preferred embodiments of the present invention will now be described by way of example only and with reference to the accompanying drawings, in which:

FIG. 1 shows a hierarchy of medical image records that can be manipulated in accordance with the disclosed subject matter.

FIG. 2 shows the architecture of a system for manipulating images, in accordance with the disclosed subject matter.

FIG. 3 shows mammogram images in accordance with the disclosed subject matter.

FIGs. 4A-4D show mammogram image processing to identify a chest wall line in accordance with the disclosed subject matter.

FIGs. 5A-5C show mammogram image processing to identify a nipple location in accordance with the disclosed subject matter.

FIG. 6 shows a mammogram with a posterior nipple line (PNL) and region of interest, in accordance with the disclosed subject matter.

FIG. 7 shows the mammogram of FIG. 6 showing a distance d, in accordance with the disclosed subject matter.

FIG. 8 shows a mammogram with a CC projection of the breast in the mammogram of FIG. 6, in accordance with the disclosed subject matter.

FIG. 9 shows the mammogram of FIG. 8 showing an area-of-interest marker, in accordance with he disclosed subject matter.

FIG. 10 shows a mammogram, in accordance with the disclosed subject matter.

FIG. 11 shows a mammogram of the same breast as FIG. 10 taken about two years earlier, in accordance with the disclosed subject matter.

FIG. 12 shows a flow chart illustrating a method for marking a region of interest in a mammogram, in accordance with the disclosed subject matter.

FIG. 13 shows a flow chart illustrating a method for registering two mammogram images, in accordance with the disclosed subject matter.

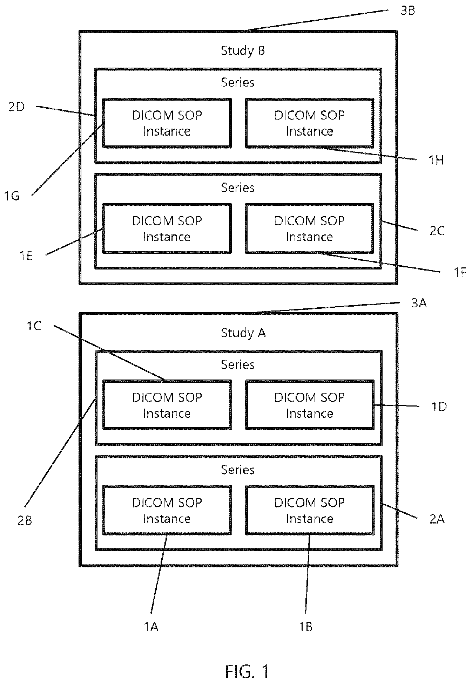

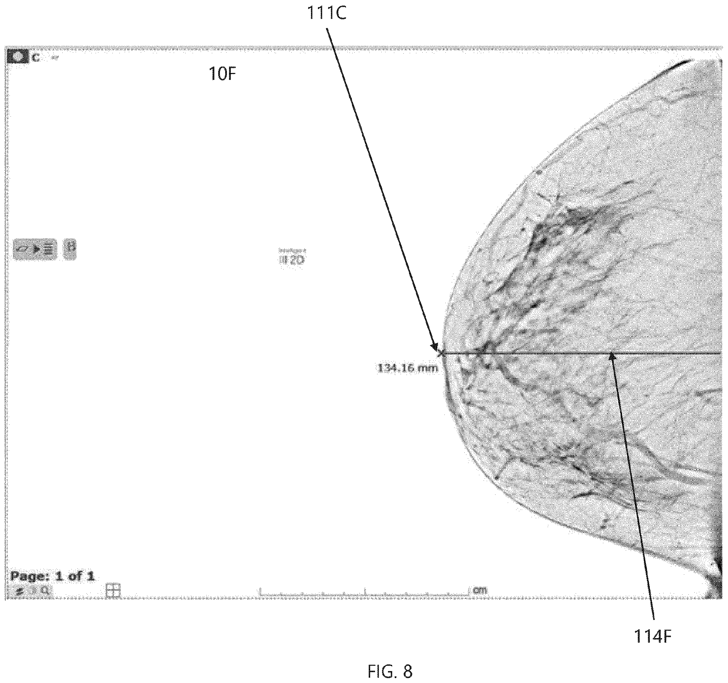

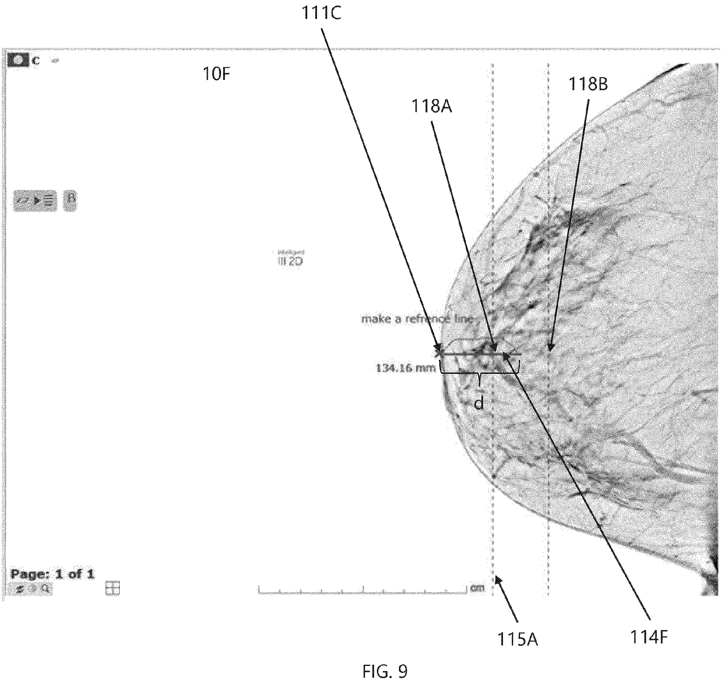

Reference will now be made in detail to various exemplary embodiments of the present invention, exemplary embodiments of which are illustrated in the accompanying drawings. For purpose of illustration and not limitation, the systems and method are described herein with respect to manipulating images, and particularly, digital medical images (also referred to as "medical images"), such as DICOM images, and more specifically, mammograms. However, the methods and systems described herein can be used for manipulating any digital image. As used in the description and the appended claims, the singular forms, such as "a," "an," "the," and singular nouns, are intended to include the plural forms as well, unless the context clearly indicates otherwise. Accordingly, as used herein, the term medical image can refer to one medical image, or a plurality of medical images. For example, and with reference to FIG. 1 for purpose of illustration and not limitation, as referred to herein a medical image record can include a single DICOM Service-Object Pair ("SOP") Instance (also referred to as "DICOM Instance" "DICOM image" and "image") 1 (e.g., 1A-1H), one or more DICOM SOP Instances 1 in one or more Series 2 (e.g., 2A-D), one or more Series 2 inside one or more Studies 3 (e.g., 3A, 3B), and one or more Studies 3. A DICOM SOP Instance 1 (e.g., 1A-1H) can be mammogram. Referring to FIGs. 1-11 for purpose of illustration and not limitation, the disclosed system 100 can be configured to manipulate images. For example, system 100 can be configured for marking regions of interest in mammograms and/or registering mammograms, such as DICOM images (e.g., 1A-1H). The system 100 can include one or more computing devices defining a server 30 and user workstation 60. The user workstation 60 can be coupled to the server 30 by a network. The network, for example, can be a Local Area Network ("LAN"), a Wireless LAN ("WLAN"), a virtual private network ("VPN"), or any other network that allows for any radio frequency or wireless type connection. For example, other radio frequency or wireless connections can include, but are not limited to, one or more network access technologies, such as Global System for Mobile communication ("GSM"), Universal Mobile Telecommunications System ("UMTS"), General Packet Radio Services ("GPRS"), Enhanced Data GSM Environment ("EDGE"), Third Generation Partnership Project ("3GPP") Technology, including Long Term Evolution ("LTE"), LTE-Advanced, 3G technology, Internet of Things ("IOT"), fifth generation ("5G"), or new radio ("NR") technology. Other examples can include Wideband Code Division Multiple Access ("WCDMA"), Bluetooth, IEEE 802.11b/g/n, or any other 802.11 protocol, or any other wired or wireless connection.

Workstation 60 can take the form of any known client device. For example, workstation 60 can be a computer, such as a laptop or desktop computer, a personal data or digital assistant ("PDA"), or any other user equipment or tablet, such as a mobile device or mobile portable media player. Server 30 can be a service point which provides processing, database, and communication facilities. For example, the server 30 can include dedicated rack-mounted servers, desktop computers, laptop computers, set top boxes, integrated devices combining various features, such as two or more features of the foregoing devices, or the like. Server 30 can vary widely in configuration or capabilities, but can include one or more processors, memory, and/or transceivers. Server 30 can also include one or more mass storage devices, one or more power supplies, one or more wired or wireless network interfaces, one or more input/output interfaces, and/or one or more operating systems.

A user can be any person authorized to access workstation 60 and/or server 30, including a health professional, medical technician, researcher, or patient. In some embodiments a user authorized to use the workstation 60 and/or communicate with the server 30 can have a username and/or password that can be used to login or access workstation 60 and/or server 30. Workstation 60 can include GUI 65, memory 61, processor 62, transceiver 63, and input/output 64. Medical image records (such as mammograms 10) received by workstation 60 can be processed using one or more processors 62. Processor 62 can be any hardware or software used to execute computer program instructions. These computer program instructions can be provided to a processor of a general purpose computer to alter its function to a special purpose, a special purpose computer, application-specific integrated circuit ("ASIC"), or other programmable digital data processing apparatus, such that the instructions, which execute via the processor of the workstation 60 or other programmable data processing apparatus, implement the functions/acts specified in the block diagrams or operational block or blocks, thereby transforming their functionality in accordance with embodiments herein. The processor 62 can be a portable embedded micro-controller or micro-computer. For example, processor 62 can be embodied by any computational or data processing device, such as a central processing unit ("CPU"), digital signal processor ("DSP"), ASIC, programmable logic devices ("PLDs"), field programmable gate arrays ("FPGAs"), digitally enhanced circuits, or comparable device or a combination thereof. The processor 62 can be implemented as a single controller, or a plurality of controllers or processors. The input/output 64 can be any suitable input/output, for example, a mouse or keyboard. In accordance with the disclosed subject matter, input/output 64 can be integrated with GUI 65 in the form of a touchscreen. Input/output 64 can be implemented as a single input/output 64 or a plurality of input/outputs 64.

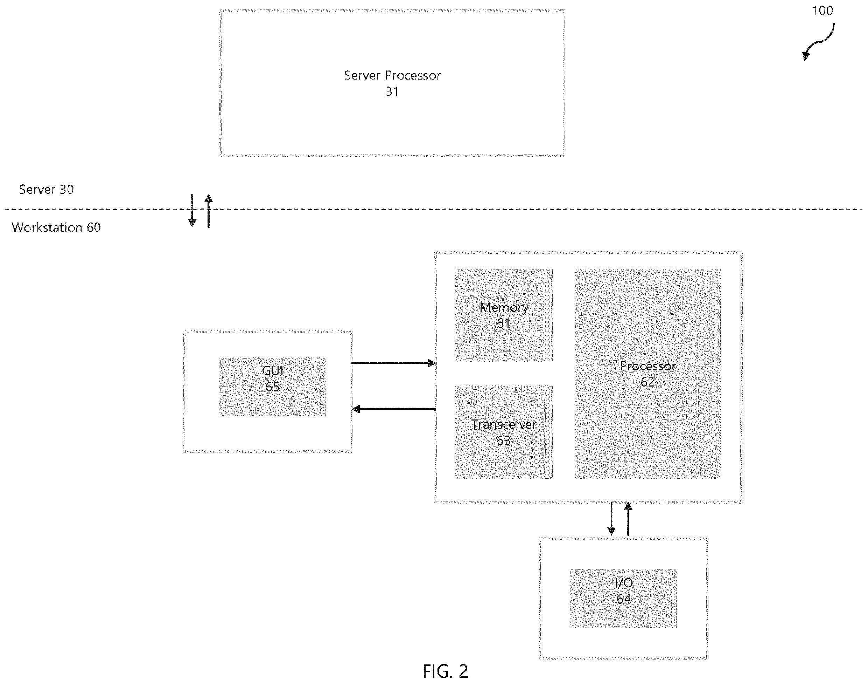

Workstation 60 can send and receive medical image records (such as mammograms 10) from server 30 using transceiver 63. Transceiver 63 can, independently, be a transmitter, a receiver, or both a transmitter and a receiver, or a unit or device that can be configured both for transmission and reception. In other words, transceiver 63 can include any hardware or software that allows workstation 60 to communicate with server 30. Transceiver 63 can be either a wired or a wireless transceiver. When wireless, the transceiver 63 can be implemented as a remote radio head which is not located in the device itself, but in a mast. While FIG. 2 only illustrates a single transceiver 63, workstation 60 can include one or more transceivers 63. Memory 61 can be a non-volatile storage medium or any other suitable storage device, such as a non-transitory computer-readable medium or storage medium. For example, memory 61 can be a random-access memory ("RAM"), read-only memory ("ROM"), hard disk drive ("HDD"), erasable programmable read-only memory ("EPROM"), electrically erasable programmable read-only memory ("EEPROM"), flash memory or other solid-state memory technology. Memory 61 can also be a compact disc read-only optical memory ("CD-ROM"), digital versatile disc ("DVD"), any other optical storage, magnetic cassettes, magnetic tape, magnetic disk storage or other magnetic storage devices, or any other physical or material medium which can be used to tangibly store the desired information or data or instructions and which can be accessed by a computer or processor. Memory 61 can be either removable or non-removable.

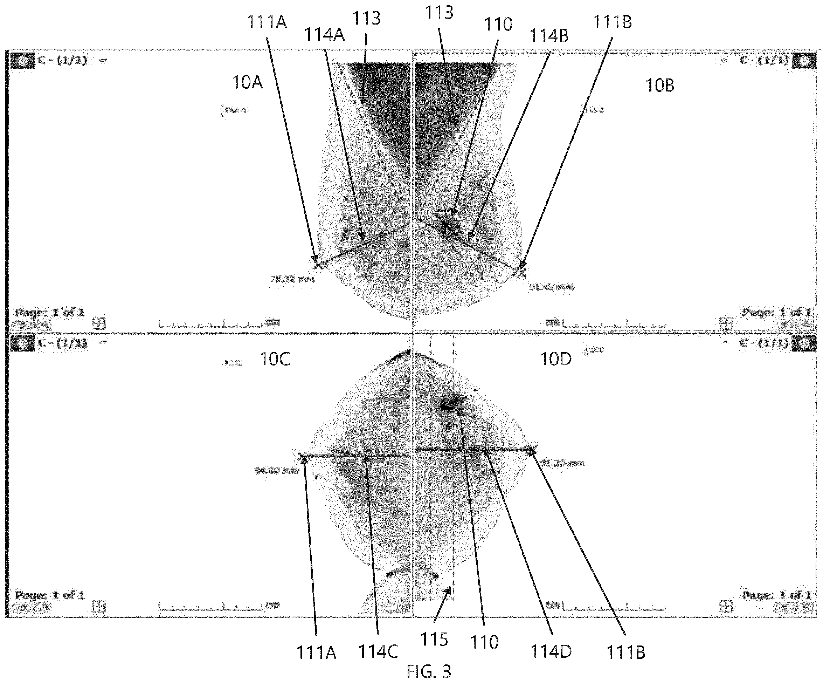

Referring to FIG. 3, for purpose of illustration and not limitation, four mammograms 10A-10D are shown. The mammograms 10A-10D can be DICOM images (e.g., 1J). Mammogram 10A is a right breast MLO image; Mammogram 10B is a left breast MLO image; Mammogram 10C is a CC image of the right breast of Mammogram 10A; Mammogram 10D is a CC image of the left breast of Mammogram 10B. Each image comes from the same patient (anonymous female born in 1964). Mammograms 10A and 10C include left nipple 111A and Mammograms 10B and 10C include right nipple 111B. The locations of the left nipple 111A and right nipple 111B can be identified by a user (e.g., by pointing and clicking on the location), by a machine learning algorithm (e.g., by training to identify the location of the nipple 111 (e.g., 111A, 111B) on a series of images and then automatically identifying the location), by a combination of a machine learning algorithm and user input, or other suitable means.

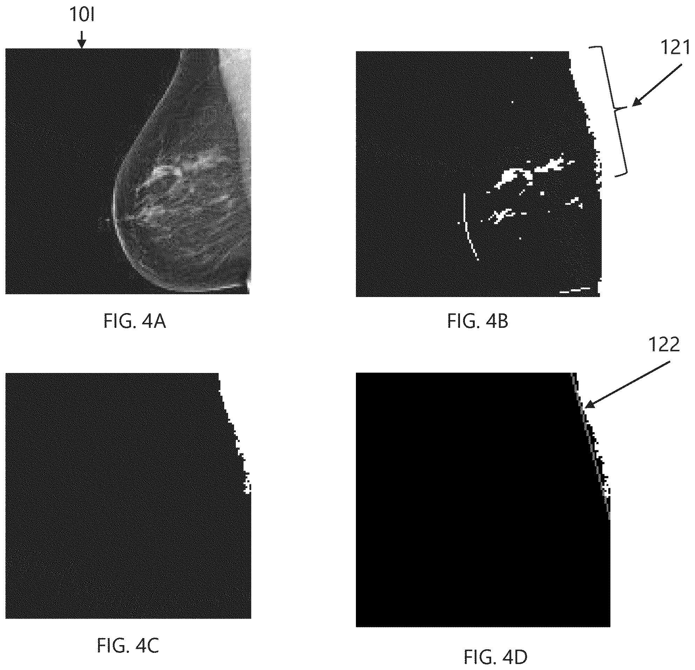

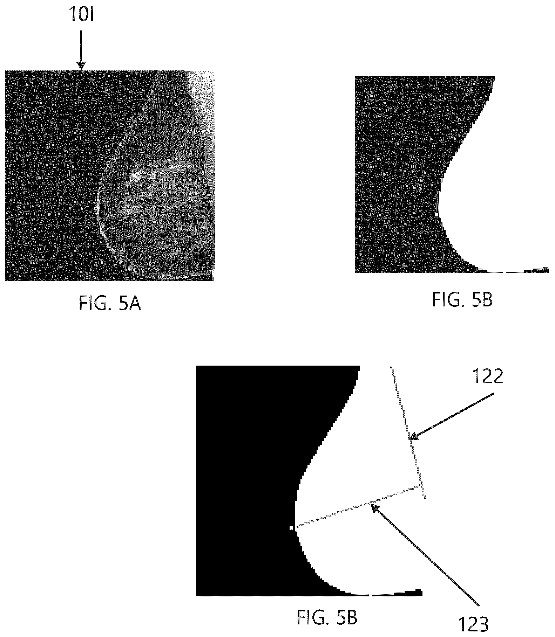

Mammograms 10A and 10B show chest wall 113 (marked as a dotted line). The chest wall is always perpendicular to one side in a CC image (mammograms 10C and 10D). The chest wall 113 location can also be identified by a user (e.g., by pointing and clicking, for example on two spots to identify a chest wall line), by a machine learning algorithm, or other suitable means. As an example, and with reference to FIGs. 4A-4D for purpose of illustration and not limitation, the chest wall can be identified based on pixel values. For example, white areas above a specific pixel threshold (for example, 128) of mammogram 10I can be identified. A black and white image, for example as shown in FIG. 4B can be generated based on the pixels above the threshold. White pixels next to a side or corner, for example region 121, can be identified and a third image, as shown in FIG. 4C, can be generated. A line 122 (FIG. 4D) can be generated. For example, line 122 can be the nearest to the center of the image straight line to include all white pixels. As another example, the line 122 can be the longest straight line to include all white pixels. Line 122 can represent the chest wall 113. Additionally or alternatively, pixels can be used to identify the nipple location. With reference to FIGs. 5A-5C for purpose of illustration and not limitation, the nipple location can be identified based on pixel values. For example, white area above a specific pixel padding value (for example, 0) of mammogram 10I can be identified. A black and white image, for example as shown in FIG. 5B can be generated based on the pixels above the padding value. From the breast area and the chest line 122 the farthest point can be identified (for example using line 123), and that point can be used as the nipple location. Mammograms 10 each include a posterior nipple line (PNL) 114 (e.g., 114A-114D). The PNL 114 extends through the respective nipple 111 and perpendicular to the chest wall 113 (or the side of the image in mammograms with a CC projection). Mammograms 10B and 10D have region of interest 110. Region of interest 110 can indicate a potential tumor, or other anatomical feature. The region of interest 110A can be identified by a user (e.g., by pointing and clicking, for example on two spots to identify a chest wall line), by a machine learning algorithm, or other suitable means. Mammogram 10D also includes a region-of-interest marker 115. Although illustrated as two dotted lines, region-of-interest marker 115 can be any suitable marking, such as a shaded region, a singled dotted line, box, or circle. Furthermore, more than one region-of-interest marker 115 can be provided on a mammogram.

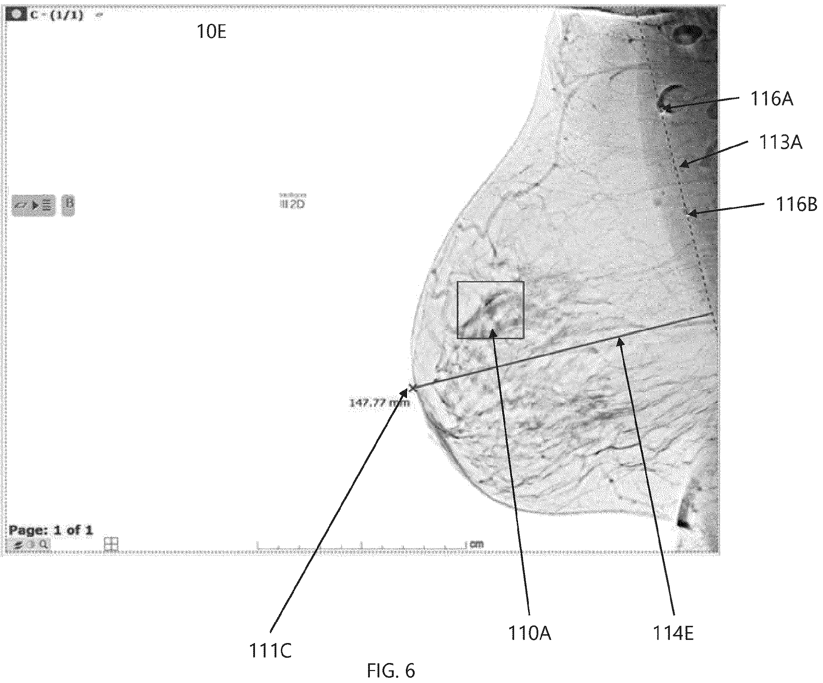

Referring to FIGs. 6-9 for purpose of illustration and not limitation, mammogram 10E is an MLO projection and mammogram 10F is a CC projection of the same breast shown in mammogram 10E. In operation, system 100 can provide a region-of-interest marker 115A in mammogram 10F based on the identification of the region of interest 110A in mammogram 10E. Mammogram 10E can be displayed on GUI 65. Initially, the location of nipple 111C can be identified. For example, a user can click on the location of the nipple 111C. The location of chest wall 113A can also be identified. For example, a user can click on at least two points 116A, 116B along the chest wall. These points 116A, 116B can be used to identify the location of the chest wall 113A, which can be treated as a straight line through points 116A, 116B. PNL 114E can be identified by drawing a line through the nipple 111C and perpendicular to the chest wall 113A. Region of interest 110A can also be identified, for example, by the user clicking on the region of interest.

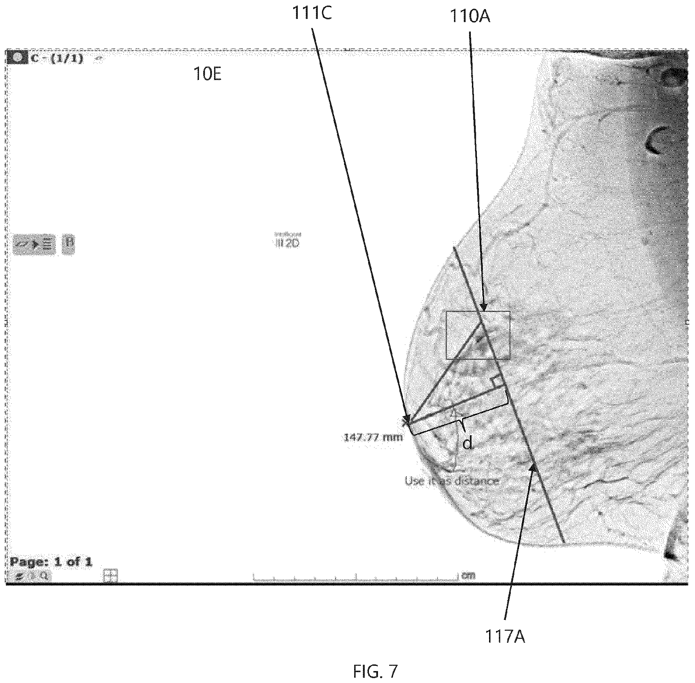

As shown in FIGs. 6 and 7, for purpose of illustration and not limitation, system 100 can identify reference line 117A in mammogram 10E. Reference line 117A can extend through the region of interest 110A and perpendicular to the PNL 114E. A triangle can be formed with vertices at the nipple 111C, the region of interest 110A, and the intersection of the PNL 114E and the reference line 117A. System 100 can determine distance, d, extending from the nipple 111C to the reference line 117A along the PNL 114E. For example, the distance, d, can be determined using the formula: Distance = (PNL unit vector * (region-of-interest position - nipple 111C position). The PNL unit vector can be determined as follows: The chest wall 113A can be defined as a unit vector calculated by two points 116A $(x_1, y_1)$ and 116B $(x_2, y_2)$ on the chest wall 113A line. A distance can be calculated according to equation 1:

$$(1)\ d = \sqrt{(x_1 - x_2) * (x_1 - x_2) + (y_1 - y_2) * (y_1 - y_2)}$$

The chest wall unit vector can be $((x_1\text{-}x_2)/d, (y_1\text{-}y_2)/d)$. The PNL unit vector can be 90 degrees from the chest wall unit vector and can be $((y_1-y_2)/d, -(x_1,x_2)/d)$. Although a particular method of determining distance d, any suitable method can be used to determine distance d. Reference line 117A can be displayed on mammogram 10E and its location can be adjusted, for example, by a user dragging reference line 117A along PNL 114E.

Mammogram 10F can be displayed on GUI 65. Mammograms 10E and 10F can be displayed on GUI 65 simultaneously (e.g., side-by-side) or in series. The location of nipple 111C can be identified in mammogram 10F in a similar method as described above. PNL 114F can be identified by drawing a line through the nipple 111C and perpendicular to the side of mammogram 10E. System 100 can determine the distance d along PNL 114F. System 100 can further determine two points 118A, 118B along PNL 114F equidistant from the distance d along PNL 114F. Region-of-interest marker 115A (shown as two dotted lines) can be provided on mammogram 10F extending perpendicular to PNL 114F from points 118A, 118B. Accordingly, a user would understand that the region of interest 110 is located within the dotted lines of the region-of-interest marker 115A. Although shown as two dotted lines, region-of-interest marker 115A can be provided in any suitable manner by extending perpendicularly away from PNL 114F a distance d from the nipple 111C. Because the algorithm treats the direction of the mammogram as perpendicular to the PNL 114, the region-of-interest marker 115 can be more accurate than traditional arcs drawn from the nipple 111. When the image is a synthesized 2D image with map information, a rectangle can be used as the region-of-interest marker 115. Map information can store depth or frame information for each pixel. From the user clicked position, the system 100 can convert to the breast depth information. From depth information, the system 100 can calculate the target position and show a region-of-interest marker 115 (such as a rectangle).

As described above, the system 100 provides a region-of-interest marker 115 in a mammogram 10 taken from a different projection. Although described as identifying the region of interest 110 on the MLO image and providing the region-of-interest marker 115 on the CC image, the reverse can be performed. In accordance with the disclosed subject matter, system 100 can provide a region-of-interest marker 115 in a second mammogram 10 take from the same projection but at a different date and time. A region-of-interest marker 115 can be provided in one mammogram (e.g., 10H) based on the identification of a region of interest 110 in the manner described above. Although described as determining distance d for placing the region-of-interest marker 115, other suitable methods can be used. As an example, ratios can be used to address changes in breast area or pressing. For example, a ratio of distance d to the total length of PNL 114 can be determined. The location of the region-of-interest marker 115 in the second mammogram 10 can then be determined based on the total length of the PNL 114 in the second mammogram 10 and the ratio.

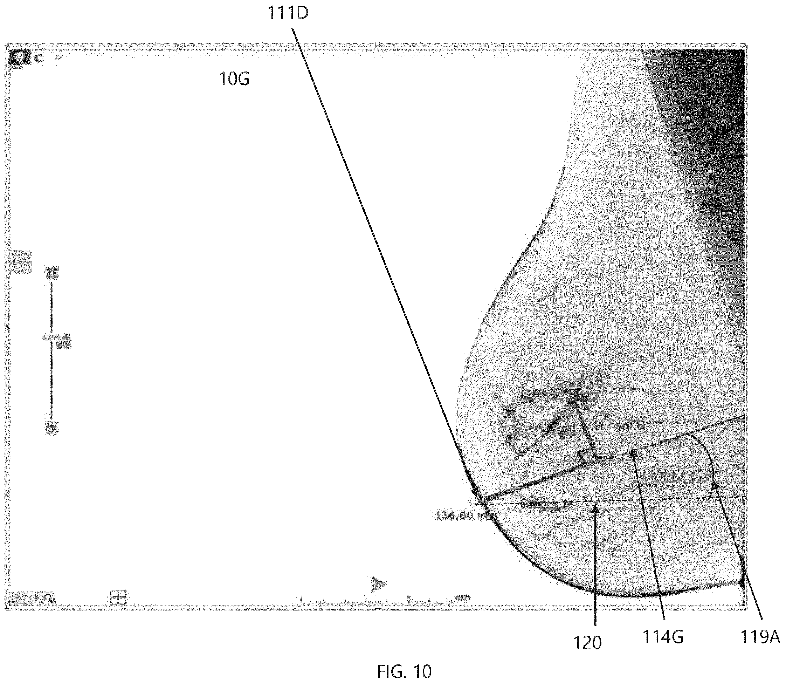

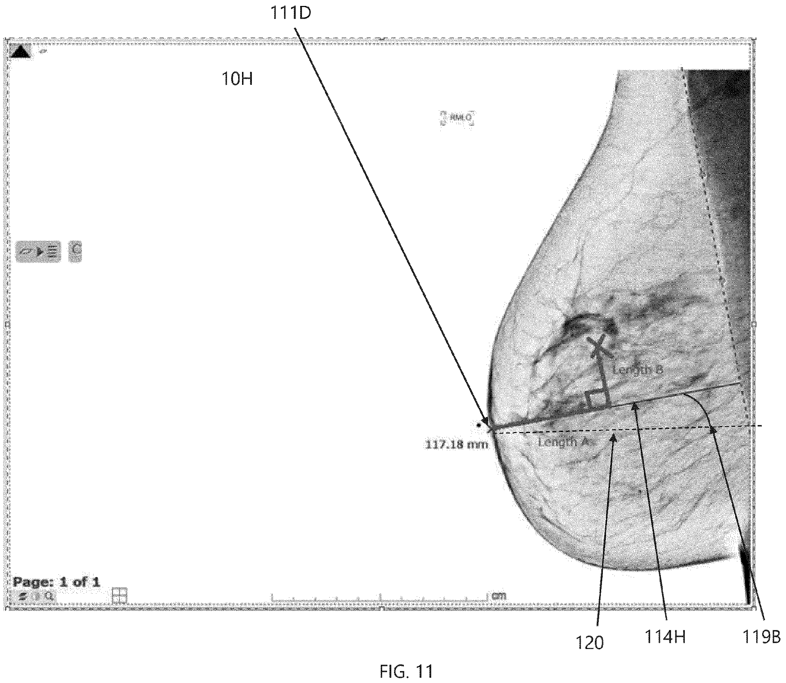

In accordance with the disclosed subject matter, system 100 can register first and second mammograms 10 taken from the same projection but at a different date and time. Referring to FIGs. 10 and 11 for purpose of illustration and not limitation, a mammogram 10G is an MLO projection and mammogram 10H is an MLO projection of the same breast taken about two years earlier. The system 100 can determine a location of nipple 111D and can draw PNL 114G in mammogram 10G as described above. The system 100 can likewise determine the location of nipple 111D and PNL 114H in mammogram 10H. System 100 can register the two mammograms by shifting mammogram 10H to align nipple 111D in each mammogram 10G, 10H. For example, system 100 can shift mammogram 10H vertically by a difference between the vertical coordinate of nipple 111D in mammogram 10H and the vertical coordinate of nipple 111D in mammogram 10G. Likewise, mammogram 10H can be shifted horizontally by a difference between the horizontal coordinate of nipple 111D in mammogram 10H and the horizontal coordinate of nipple 111D in mammogram 10G. Mammogram 10H can be rotated by a difference between an angle 119B of the PNL 114H and an angle 119A of PNL 114G. For example, the angle 119A can be determined as compared to horizontal 120. In the respective mammograms 10G, 10H. Such a process can allow a user to align mammograms 10 more easily. Mammograms 10G and 10H can be displayed on GUI 65 simultaneously (e.g., side-by-side) or in series.

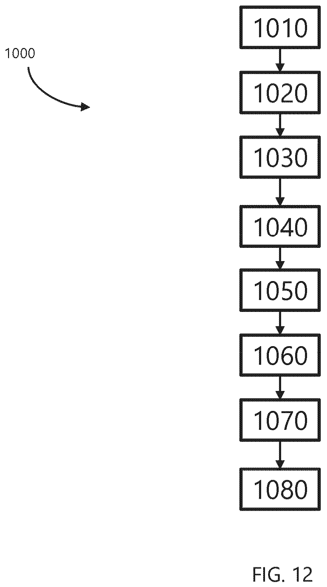

FIG. 12 illustrates an example method 1000 for marking a region of interest in a mammogram.

The method can begin at step 1010, where the method includes receiving a first mammogram of a patient, the first mammogram comprising a first nipple tip and being a first projection. At step 1020, the method can include identifying a first posterior nipple line in the first mammogram. At step 1030, the method can include identifying a region of interest in the first mammogram. At step 1040, the method can include identifying a reference line in the first mammogram, the reference line being perpendicular to the first posterior nipple line and extending from the posterior nipple line through the region of interest. At step 1050, the method can include calculating a distance, d, the distance d extending from the first nipple tip to the reference line along the first posterior nipple line. At step 1060, the method can include receiving a second mammogram of the patient, the second mammogram comprising a second nipple tip and being a second projection. At step 1070, the method can include identifying a second posterior nipple line in the second mammogram. At step 1080, the method can include providing, on the second mammogram, a region-of-interest marker, the region-of-interest marker extending perpendicular from the second posterior nipple line the distance d from the second nipple tip along the second posterior nipple line. In accordance with the disclosed subject matter, the method can repeat one or more steps of the method of FIG. 12, where appropriate. Although this disclosure describes and illustrates particular steps of the method of FIG. 12 as occurring in a particular order, this disclosure contemplates any suitable steps of the method of FIG. 12 occurring in any suitable order. Moreover, although this disclosure describes and illustrates an example method for marking a region of interest in a mammogram including the particular steps of the method of FIG. 12, this disclosure contemplates any suitable method for marking a region of interest in a mammogram including any suitable steps, which can include all, some, or none of the steps of the method of FIG. 12, where appropriate. Furthermore, although this disclosure describes and illustrates particular components, devices, or systems carrying out particular steps of the method of FIG. 12, this disclosure contemplates any suitable combination of any suitable components, devices, or systems carrying out any suitable steps of the method of FIG. 12.

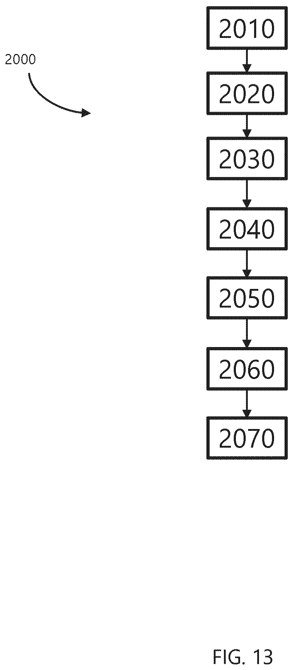

FIG. 13 illustrates an example method 2000 for registering two mammogram images. The method can begin at step 2010, where the method includes receiving a first mammogram of a patient, the first mammogram comprising a first nipple tip having a first vertical coordinate and a first horizontal coordinate. At step 2020, the method can include identifying a first posterior nipple line in the first mammogram, the first posterior nipple line having a first angle. At step 2030, the method can include receiving a second mammogram of the patient, the second mammogram comprising a second nipple tip having a second vertical coordinate and a second horizontal coordinate. At step 2040, the method can include identifying a second posterior nipple line in the second mammogram, the second posterior nipple line having a second angle. At step 2050, the method can include shifting the second mammogram vertically by a difference between the second vertical coordinate and the first vertical coordinate. At step 2060, the method can include shifting the second mammogram horizontally by a difference between the second horizontal coordinate and the first horizontal coordinate. At step 2070, the method can include rotating the second mammogram by a difference between the second angle and the first angle. In accordance with the disclosed subject matter, the method can repeat one or more steps of the method of FIG. 13, where appropriate. Although this disclosure describes and illustrates particular steps of the method of FIG. 13 as occurring in a particular order, this disclosure contemplates any suitable steps of the method of FIG. 13 occurring in any suitable order. Moreover, although this disclosure describes and illustrates an example method for registering two mammogram images including the particular steps of the method of FIG. 13, this disclosure contemplates any suitable method for registering two mammogram images including any suitable steps, which can include all, some, or none of the steps of the method of FIG. 13, where appropriate. Furthermore, although this disclosure describes and illustrates particular components, devices, or systems carrying out particular steps of the method of FIG. 13, this disclosure contemplates any suitable combination of any suitable components, devices, or systems carrying out any suitable steps of the method of FIG. 13.

The subject matter and the operations described in this specification can be implemented in digital electronic circuitry, or in computer software, firmware, or hardware, including the structures disclosed in this specification and their structural equivalents, or in combinations of one or more of them. Embodiments of the invention described in this specification can be implemented as one or more computer programs, i.e., one or more modules of computer program instructions, encoded on computer storage medium for execution by, or to control the operation of, data processing apparatus.

A computer storage medium can be, or can be included in, a computer-readable storage device, a computer-readable storage substrate, a random or serial access memory array or device, or a combination of one or more of them. Moreover, while a computer storage medium is not a propagated signal, a computer storage medium can be a source or destination of computer program instructions encoded in an artificially-generated propagated signal. The computer storage medium also can be, or may be included in, one or more separate physical components or media (e.g., multiple CDs, disks, or other storage devices).

The term "processor" encompasses all kinds of apparatus, devices, and machines for processing data, including by way of example a programmable processor, a computer, a system on a chip, or multiple ones, or combinations, of the foregoing. The apparatus can include special purpose logic circuitry, e.g., an FPGA or an ASIC. The apparatus also can include, in addition to hardware, code that creates an execution environment for the computer program in question, e.g., code that constitutes processor firmware, a protocol stack, a database management system, an operating system, a cross-platform runtime environment, a virtual machine, or a combination of one or more of them. The apparatus and execution environment can realize various different computing model infrastructures, such as web services, distributed computing and grid computing infrastructures.

A computer program (also known as a program, software, software application, script, or code) can be written in any form of programming language, including compiled or interpreted languages, declarative or procedural languages, and it can be deployed in any form, including as a stand-alone program or as a module, component, subroutine, object, or other unit suitable for use in a computing environment. A computer program can, but need not, correspond to a file in a file system. A program can be stored in a portion of a file that holds other programs or data (e.g., one or more scripts stored in a markup language document), in a single file dedicated to the program in question, or in multiple coordinated files (e.g., files that store one or more modules, sub-programs, or portions of code). A computer program can be deployed to be executed on one computer or on multiple computers that are located at one site or distributed across multiple sites and interconnected by a communication network.

The processes and logic flows described in this specification can be performed by one or more programmable processors executing one or more computer programs to perform actions by operating on input data and generating output. The processes and logic flows can also be performed by, and apparatus can also be implemented as, special purpose logic circuitry, e.g., an FPGA or an ASIC.

Processors suitable for the execution of a computer program can include, by way of example and not by way of limitation, both general and special purpose microprocessors. Devices suitable for storing computer program instructions and data can include all forms of non-volatile memory, media and memory devices, including by way of example but not by way of limitation, semiconductor memory devices, e.g., EPROM, EEPROM, and flash memory devices; magnetic disks, e.g., internal hard disks or removable disks; magneto-optical disks; and CD-ROM and DVD-ROM disks. The processor and the memory can be supplemented by, or incorporated in, special purpose logic circuitry.

Additionally, as described above in connection with certain embodiments, certain components can communicate with certain other components, for example via a network, e.g., a local area network or the internet. To the extent not expressly stated above, the disclosed subject matter is intended to encompass both sides of each transaction, including transmitting and receiving. One of ordinary skill in the art will readily understand that with regard to the features described above, if one component transmits, sends, or otherwise makes available to another component, the other component will receive or acquire, whether expressly stated or not.

In addition to the specific embodiments claimed below, the present application also incorporates other embodiments having any other possible combination of the dependent features claimed below and those disclosed above. As such, the particular features presented in the dependent claims and disclosed above can be combined with each other in other possible combinations. Thus, the foregoing description of specific embodiments of the invention has been presented for purposes of illustration and description. It is not intended to be exhaustive or to limit the invention to those embodiments disclosed.

It will be apparent to those skilled in the art that various modifications and variations can be made in the method and system of the present invention without departing from the scope of the invention as defined by the appended claims. Thus, it is intended that the invention includes modifications and variations that are within the scope of the appended claims.

The following clauses set out features of the invention which may or may not presently be claimed but which may form the basis for future amendments and/or divisional applications:

1. A system (100) comprising: one or more processors (62); and a non-transitory memory (61) coupled to the processors comprising instructions executable by the processors, the processors being operable when executing the instructions to:

receive a first mammogram (10E) of a patient, the first mammogram comprising a first nipple tip (111C) and being a first projection;
identify a first posterior nipple line (114E) in the first mammogram;
identify a region of interest (110A) in the first mammogram;
identify a reference line (117A) in the first mammogram, the reference line being perpendicular to the first posterior nipple line and extending from the first posterior nipple line through the region of interest;
calculate a distance, d, the distance d extending from the first nipple tip to the reference line along the first posterior nipple line;
receive a second mammogram (10F) of the patient, the second mammogram comprising a second nipple tip (111C) and being a second projection;
identify a second posterior nipple line (114F) in the second mammogram; and
provide, on the second mammogram, a region-of-interest marker (115A), the region-of-interest marker extending perpendicular from the second posterior nipple line the distance d from the second nipple tip along the second posterior nipple line.

2. A method (1000) for marking a region of interest (110) in a mammogram (10), comprising:

receiving (1010) a first mammogram (10E) of a patient, the first mammogram comprising a first nipple tip (111C) and being a first projection;
identifying (1020) a first posterior nipple line (114E) in the first mammogram;
identifying (1030) a region of interest (110A) in the first mammogram;
identifying (1040) a reference line (117A) in the first mammogram, the reference line being perpendicular to the first posterior nipple line and extending from the first posterior nipple line through the region of interest;
calculating (1050) a distance, d, the distance d extending from the first nipple tip to the reference line along the first posterior nipple line;
receiving (1060) a second mammogram (10F) of the patient, the second mammogram comprising a second nipple tip (111C) and being a second projection;

identifying (1070) a second posterior nipple line (114F) in the second mammogram; and

providing (1080), on the second mammogram, a region-of-interest marker (115A), the region-of-interest marker extending perpendicular from the second posterior nipple line the distance d from the second nipple tip along the second posterior nipple line.

3. The system (100) of clause 1 or the method (1000) of clause 2, wherein the first posterior nipple line (114E) is perpendicular to a chest wall (113).

4. The system (100) of clause 1 or 3, or the method (1000) of clause 2 or 3, wherein the second posterior nipple line (114F) is perpendicular to a or the chest wall (113).

5. The system (100) of clause 3 or 4, or the method (1000) of clause 3 or 4, further comprising receiving a user input identifying the chest wall (113).

6. The system (100) of any of clauses 1 or 3 to 5, or the method (1000) of any of clauses 2 to 5, wherein the first projection comprises one of a craniocaudal view and a mediolateral oblique view;

optionally wherein the second projection comprises the other of the craniocaudal view and the mediolateral oblique view.

7. The system (100) of any of clauses 1 or 3 to 6, or the method (1000) of any of clauses 2 to 6, further comprising receiving a user input indicating a location of the first nipple tip (111C).

8. The system (100) of any of clauses 1 or 3 to 7, or the method (1000) of any of clauses 2 to 7, further comprising receiving a user input indicating a location of the second nipple tip (111C).

9. A system (100) comprising: one or more processors (62); and a non-transitory memory (61) coupled to the processors comprising instructions executable by the processors, the processors being operable when executing the instructions to:

receive a first mammogram (10G) of a patient, the first mammogram comprising a first nipple tip (111D) having a first vertical coordinate and a first horizontal coordinate;

identify a first posterior nipple line (114G) in the first mammogram, the first posterior nipple line having a first angle (119A);

receive a second mammogram (10H) of the patient, the second mammogram comprising a second nipple tip (111D) having a second vertical coordinate and a second horizontal coordinate;

identify a second posterior nipple line (114H) in the second mammogram, the second posterior nipple line having a second angle (119B);

shift the second mammogram vertically by a first difference between the second vertical coordinate and the first vertical coordinate;

shift the second mammogram horizontally by a second difference between the second horizontal coordinate and the first horizontal coordinate; and

rotate the second mammogram by a third difference between the second angle and the first angle.

10. A method (2000) for registering two mammogram images (10), comprising:

receiving (2010) a first mammogram (10G) of a patient, the first mammogram comprising a first nipple tip (111D) having a first vertical coordinate and a first horizontal coordinate;

identifying (2020) a first posterior nipple line (114G) in the first mammogram, the first posterior nipple line having a first angle (119A);

receiving (2030) a second mammogram (10H) of the patient, the second mammogram comprising a second nipple tip (111D) having a second vertical coordinate and a second horizontal coordinate;

identifying (2040) a second posterior nipple line (114H) in the second mammogram, the second posterior nipple line having a second angle (119B);

shifting (2050) the second mammogram vertically by a first difference between the second vertical coordinate and the first vertical coordinate;

shifting (2060) the second mammogram horizontally by a second difference between the second horizontal coordinate and the first horizontal coordinate; and

rotating (2070) the second mammogram by a third difference between the second angle and the first angle.

11. The system (100) of clause 9 or the method (2000) of clause 10, wherein the first posterior nipple line (114G) is perpendicular to a chest wall (113);

optionally comprising receiving a user input identifying the chest wall (113).

12. The system (100) of clause 9 or 11, or the method (2000) of clause 10 or 11, wherein the second posterior nipple line (114H) is perpendicular to a chest wall (113);

optionally comprising receiving a user input identifying the chest wall (113).

13. The system (100) of clause 9, 11 or 12, or the method (2000) of clause 10, 11 or 12, wherein the first mammogram (10G) comprises a craniocaudal view and the second mammogram (10H) comprising a craniocaudal view.

14. The system (100) of any of clauses 9 or 11 to 13, or the method (2000) of any of clauses 10 to 13, wherein the first mammogram (10G) comprises a mediolateral oblique view and the second mammogram (10H) comprises a mediolateral oblique view.

15. The system (100) of any of clauses 9 or 11 to 14, or the method (2000) of any of clauses 10 to 14, further comprising receiving a user input indicating a location of the first nipple tip (111D); and/or

comprising receiving a user input indicating a location of the second nipple tip (111D).

**Claims**

1. A system (100) comprising: one or more processors (62); and a non-transitory memory (61) coupled to the processors comprising instructions executable by the processors, the processors being operable when executing the instructions to:

   receive a first mammogram (10G) of a patient, the first mammogram comprising a first nipple tip (111D) having a first vertical coordinate and a first horizontal coordinate;
   identify a first posterior nipple line (114G) in the first mammogram, the first posterior nipple line having a first angle (119A);
   receive a second mammogram (10H) of the patient, the second mammogram comprising a second nipple tip (111D) having a second vertical coordinate and a second horizontal coordinate;
   identify a second posterior nipple line (114H) in the second mammogram, the second posterior nipple line having a second angle (119B);
   shift the second mammogram vertically by a first difference between the second vertical coordinate and the first vertical coordinate;
   shift the second mammogram horizontally by a second difference between the second horizontal coordinate and the first horizontal coordinate; and
   rotate the second mammogram by a third difference between the second angle and the first angle.

2. A method (2000) for registering two mammogram images (10), comprising:

   receiving (2010) a first mammogram (10G) of a patient, the first mammogram comprising a first nipple tip (111D) having a first vertical coordinate and a first horizontal coordinate;
   identifying (2020) a first posterior nipple line (114G) in the first mammogram, the first posterior nipple line having a first angle (119A);
   receiving (2030) a second mammogram (10H) of the patient, the second mammogram comprising a second nipple tip (111D) having a second vertical coordinate and a second horizontal coordinate;
   identifying (2040) a second posterior nipple line (114H) in the second mammogram, the second posterior nipple line having a second angle (119B);
   shifting (2050) the second mammogram vertically by a first difference between the second vertical coordinate and the first vertical coordinate;
   shifting (2060) the second mammogram horizontally by a second difference between the second horizontal coordinate and the first horizontal coordinate; and
   rotating (2070) the second mammogram by a third difference between the second angle and the first angle.

3. The system (100) of claim 1 or the method (2000) of claim 2, wherein the first posterior nipple line (114G) is perpendicular to a chest wall (113).

4. The system (100) of claim 3 or the method (2000) of claim 3, comprising receiving a user input identifying the chest wall (113).

5. The system (100) of claim 1, 3 or 4, or the method (2000) of claim 2, 3 or 4, wherein the second posterior nipple line (114H) is perpendicular to a chest wall (113).

6. The system (100) of claim 5 or the method (2000) of claim 5, comprising receiving a user input identifying the chest wall (113).

7. The system (100) of any of claims 1 or 3 to 6, or the method (2000) of any of claims 2 to 6, wherein the first mammogram (10G) comprises a craniocaudal view and the second mammogram (10H) comprising a craniocaudal view.

8. The system (100) of any of claims 1 or 3 to 7, or the method (2000) of any of claims 2 to 7, wherein the first mammogram (10G) comprises a mediolateral oblique view and the second mammogram (10H) comprises a mediolateral oblique view.

9. The system (100) of any of claims 1 or 3 to 8, or the method (2000) of any of claims 2 to 8, further comprising receiving a user input indicating a location of the first nipple tip (111D).

10. The system (100) of any of claims 1 or 3 to 9, or the method (2000) of any of claims 2 to 9, comprising receiving a user input indicating a location of the second nipple tip (111D).

FIG. 1

100

Server 30

Server Processor
31

Workstation 60

Memory
61

Processor
62

Transceiver
63

I/O
64

GUI
65

FIG. 2

FIG. 3

FIG. 4A

FIG. 4B

FIG. 4C

FIG. 4D

FIG. 5B

FIG. 5A

FIG. 5B

FIG. 6

EP 4 651 081 A2

FIG. 7

FIG. 8

EP 4 651 081 A2

FIG. 9

111D

10G

119A

114G

120

Length B

Length A

136.60 mm

cm

FIG. 10

FIG. 11

1000

1010 → 1020 → 1030 → 1040 → 1050 → 1060 → 1070 → 1080

FIG. 12

EP 4 651 081 A2

2000

```
┌──────────┐
│   2010   │
└────┬─────┘
     │
     ▼
┌──────────┐
│   2020   │
└────┬─────┘
     │
     ▼
┌──────────┐
│   2030   │
└────┬─────┘
     │
     ▼
┌──────────┐
│   2040   │
└────┬─────┘
     │
     ▼
┌──────────┐
│   2050   │
└────┬─────┘
     │
     ▼
┌──────────┐
│   2060   │
└────┬─────┘
     │
     ▼
┌──────────┐
│   2070   │
└──────────┘
```

FIG. 13